# EUROPEAN PATENT SPECIFICATION

(11) **EP 1 951 172 B1**
(45) Date of publication and mention of the grant of the patent: **21.11.2018**
(21) Application number: 06794784.6
(22) Date of filing: 16.10.2006
(51) Int. Cl.: A61F 5/445, A61M 27/00

(54) **ACCESS PORT FOR A MEDICAL APPLIANCE AND METHOD**
ZUGANGSÖFFNUNG FÜR EIN MEDIZINISCHES GERÄT UND VERFAHREN
ORIFICE D'ACCES POUR UN APPAREIL MEDICAL ET PROCEDE ASSOCIE

(30) Priority: 17.10.2005 GB 0521078
(43) Date of publication of application: 06.08.2008
(73) Proprietor: T.G. Eakin Limited, Comber, County Down BT23 5QY (GB)
(72) Inventor: HEWITT, Aaron, Bangor, Co Down BT19 1BG (GB)
(74) Representative: Miller, James Lionel Woolverton
(86) International application number: PCT/GB2006/003839
(87) International publication number: WO 2007/045845

(56) References cited:
- WO-A2-2005/082271
- GB-A- 2 099 308
- US-A- 3 557 790
- US-A- 4 553 967
- US-A- 5 813 409

## Description

The present invention relates to a medical bag system with an access port such as those comprising fistula bags, pouches or wound managers.

When a patient has a wound or fistula, it may be desirable to cover the wound or fistula in order to provide a barrier. Some such wounds and fistulas may be covered with a bag which may be sealed to the patient to collect material such as fluid at the wound or fistula while also providing a barrier.

US-A-6015399 discloses a small circular gas vent for a colostomy pouch which may be attached by the user to a conventional ventless colostomy pouch - this small vent is not therefore used for the therapy of the patient. This addresses a venting problem associated specifically with colostomy pouches and does not provide access for patient tissue care or inspection. The vent has a plug with a sharp point which extends below a lower flange surface of a socket of the vent for preventing buildup of material in the colostomy bag at the entrance to the vent.

GB2099308 describes a wound drainage device with a releasable access cap.

An example of a known fistula bag is the EAKIN ® F12 fistula bag which has a layer of COHESIVE ® sealing material which seals the bag to the patient and a drainage tube for emptying the bag when necessary. This bag has proved extremely successful but needs to be removed from the patient whenever the wound, fistula or otherwise troubled part of the patient requires attention.
Another known fistula bag includes a central circular access port welded to the centre thereof. The access port provides access to the part of the patient to which the bag is attached. However, the access port is by its nature in a fixed
position relative to the bag's periphery. The therapist/user has therefore limited freedom in placing the bag to provide access in the most desirable position for access port function. Inspection also always requires the port to be opened and then closed again, potentially causing patient discomfort.

GB 2099308 discloses a prior art medical bag system with a fixed therapy access port. The present invention aims to alleviate at least to a certain extent the problems of the prior art.

According to a first aspect of the present invention there is provided a therapy access port for a medical appliance for allowing therapy of a patient therethrough, the port comprising a base, an openable closure for the base, and a seal arranged to be located on the base for sealingly connecting the port to a medical appliance. The port may be connectable to the medical appliance in a user-chosen or in a therapist or end user-chosen location position.

According to a second aspect of the invention there is provided a medical bag system comprising a therapy access port for allowing therapy of a patient therethrough, the port comprising a medical bag and an access port, the access port comprising a base and an openable closure for the base, and a seal arranged to be located on the base for sealingly connecting the port to the medical bag in one of a choice of locations.

According to a further aspect of the invention there is provided an access port for a medical appliance, the port comprising a base and an openable closure for the base, the closure co-operating with an access aperture with a minimum cross-dimension over 2 cm. The access aperture may be circular, oval or elongate. The access aperture may have a maximum cross dimension greater than 4cm, for example about 7 cm to 12 cm. When the access aperture is oval, in one embodiment it may have a minimum cross dimension (along a short axis thereof) of about 4 to 6 cm and a maximum cross dimension (along a long access thereof) of about 8 to 11 cm. This configuration may provide easy hand or tool access through the access aperture for patient therapy.

According to a further aspect of the invention there is provided a medical bag system with an access port, the port comprising a base, an openable closure for the base, the closure including an inspection window. This advantageously allows a wound or the like to be inspected through the port without the need to open the port, thereby minimizing patient discomfort.

According to a further aspect of the invention there is provided a medical bag system with an access port, the port comprising a base and an openable closure for the base, the base having a lower surface arranged to be joined to a medical appliance, the closure having a lower surface spaced above the lower surface of the base. This advantageously reduces the risk of contact between the closure and structure such as a wound of a patient to which the port has been applied.
Advantageously, in accordance with these aspects of the invention, the access port may be located on the bag or appliance wherever desired. For example, a nurse may inspect the patient and then choose where to site the access port on the appliance or bag based on such observations. For example, when a nurse would consider it most appropriate to site the access port in the middle of a medical bag such a fistula bag, the access port may be so located.
Alternatively, if it appears most appropriate for access or other reasons to locate the access port near an edge of a medical bag, this may be done instead. These aspects of the invention therefore provide increased flexibility and choice ultimately leading to improved patient comfort.

A number of optional features in connection with either or both of the above aspects of the invention will now be discussed.

The bag has a membrane surface on a first side thereof, and the membrane surface may be arranged for the formation of an inspection hole therein. The port may be arranged to sealingly engage around the inspection hole. The inspection hole may be formed after the port has been applied to the appliance or bag, or before as desired. The bag may have a sealing layer on a second side thereof, opposite the first side, which is adapted to seal the bag to a substrate of a patient. The sealing layer may be a self adhesive layer.
The bag may be arranged to be mounted on a patient, for example to skin of a patient around a wound or fistula.
The base may be non-circular, for example oval. This has the substantial advantage that the base may be formed with a relatively small overall size, but the base may be provided with a central oval aperture which allows access therethrough by a hand, thereby allowing a nurse or other operator greater and more flexible access therethrough.
The base may have a generally flat base plate having an access aperture formed in or near the centre thereof. The seal may comprise an adhesive layer located adjacent the base plate. This may provide a convenient structure in which the base plate may be pushed on to a bag or other appliance to connect the two together in a simple way near the point of use and without requiring complicated welding apparatus as in the prior art.

The adhesive layer may be located upon and cover an area of, or all of a lower surface of, a mounting substrate, the mounting substrate being secured to the base, the adhesive layer facing away from the base plate.
The mounting substrate may include a cutting tool access aperture formed therethrough. Therefore, the mounting substrate may be cut as desired, for example by a nurse, to form an access aperture of desired size and overall configuration, for example based upon patient observations.
The adhesive layer, in a pre-use configuration, may be protected by a peel-off layer.
The mounting substrate may include indicia thereon for indicating a maximum suitable aperture dimension therefor. Accordingly, this may warn the user, such as a nurse, from making an unsuitably large aperture.
At least one of the base plate and enclosure member may include a pinch flange arranged to assist in manual closure of the port with a pinching action, and to assist in opening. Accordingly, it may not be necessary to press down upon the patient to open and/or close the access port, thereby avoiding discomfort to the patient.
The closure member may have an outer edge arranged to snap-lockingly seal on to a connector part of the base plate and the outer edge may comprise a hoop of material. A closure substrate may sealingly close the hoop. The closure substrate may be transparent. This has the advantage that the wound may be clearly inspected through the access port without opening the port: in a prior art fistula bag known to the inventor, a unitary opaque closure is provided, thereby requiring potentially uncomfortable opening thereof for inspection of the area of the patient located behind the access port.

In another embodiment, the port may comprise an access bag and the base plate thereof may comprise a first flexible substrate layer thereof. This allows an access port with a lightweight construction.

The access bag may have a second flexible substrate and the flexible substrate layers may be joined at respective edges thereof. The bag may comprise a rectangular bag. The first flexible layer may have a slit formed therethrough for communication with the interior of a medical bag.

The closure may comprise at least one elongate component arranged to releasably join the first and second flexible substrates together therealong for releasably closing the access bag.

According to a further aspect of the invention there is provided a method of assembling a medical appliance system having a medical appliance and an access port, the method comprising selecting a desired location for mounting the access port on the appliance and mounting the access port on the appliance in the desired location with an adhesive component. This has the advantage that the system may be assembled as desired by an individual near the point of use. However, this need not necessarily be the case and the whole method may if desired be completed in some embodiments earlier, for example in the manufacturer's works e.g. before any details of any patients are known. The method may include providing the appliance in the form of a bag and forming a communication aperture in the bag once the port is mounted on the bag. The access port may be as set out in the first aspect of the invention. The bag system may comprise a system as in the second aspect of the invention.

The invention can be used in a method of operating a medical bag system having a medical bag and an access port, the method comprising observing a patient, selecting a location for the access port on the medical bag based upon observations of a patient, mounting the access port on the medical bag in the selected location, and mounting the system on a patient.
The access port may be mounted on the bag once the bag is on the patient.
The method may include opening the access port while the system is mounted on the patient.

According to a further aspect of the invention there is provided a medical bag system with access port, the access port having a non-circular yet curved configuration. The non-circular yet curved (for example oval or elongate) configuration may advantageously provide that an overall relatively small access port may be provided, yet one through which access by hand may be more conveniently obtained than with a circular or rectangular port.
The present invention may be carried out in various ways and two embodiments of access ports and medical bag systems in accordance with the invention will now be described by way of example with reference to the accompanying drawings, in which:
Figure 1 is a front view of a first embodiment of an access port in accordance with the present invention;
Figure 2 is a section on the line II-II';
Figure 3a is a front view of a known medical bag with a drainage tube thereof in an open extended configuration;
Figure 3b shows the bag of Figure 3a with the drainage tube closed and stowed;
Figure 4a is a side view corresponding to Figure 3a;
Figure 4b is a side view corresponding to Figure 3b;
Figure 5 shows the access port of Figure 1 attached to the bag of Figures 3 and 4;
Figure 6 shows a second embodiment of an access port in accordance with a second preferred embodiment of the present invention; and
Figure 7 is a section on the line VII-VII' in Figure 6

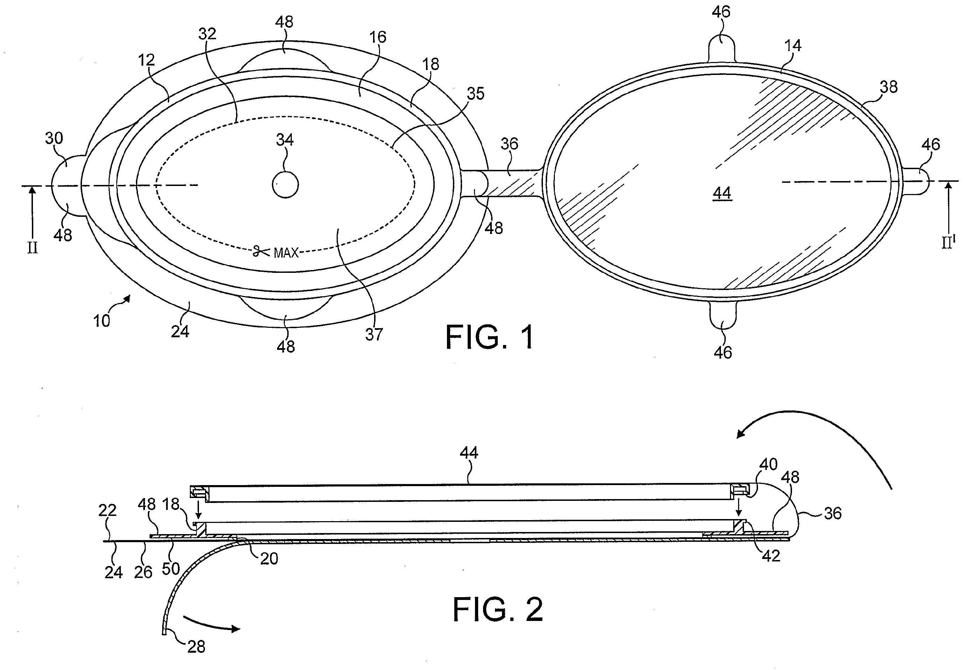

As shown in Figure 1, a first embodiment of an access port 10 in accordance with the present invention has a base 12 and closure or lid 14. The base has an oval flange 16 having an upstanding connector part or rim 18. The flange 16 is in the form of a base plate for the base 12 and is attached by adhesive or a weld 20 to a thin mounting substrate whose lower surface 24 is entirely covered with adhesive 26. A removable peel layer 28 may be removed from the mounting substrate 22 at the point of use by pulling on the peel tab 30 in order to expose the adhesive 26. Visible from the top or front of the access port is an indicia line 32 which indicates the maximum extent of any aperture which may be cut in the mounting substrate 22 (optionally by enlargement of optional aperture 34 as will be described below). The mounting substrate includes the aperture 34 formed therethrough, through which scissors or another cutting tool may be inserted in order to enlarge the aperture 34 towards the maximum extent line 32 as will be described below.

The lid 14 is connected to the base 12 by a flexible hinge 36 (or by other means). The lid 14 has an outer edge 38 having a sealing formation 40 which is arranged to sealingly connect in a releasable manner to a sealing formation 42 on the rim 18 of the base 12. The outer edge 38 is sealed by an oval window 44 made of transparent substrate material.

The sealing formations 40, 42 are designed to releasably snap lock together. Flanges 46, 48 are provided on the lid 14 and base 12 respectively for assisting in opening the lid 14 and flanges 46 and/or flanges 48 may be optionally omitted in other embodiments if desired. In other embodiments, not so many pinch flanges may be provided and indeed none may be necessary since the weld 20 only extends partially radially across the lower face 50 of the oval flange 16 such that a user may in some cases slide one or more fingers below the flange 16 for pinch closing purposes.

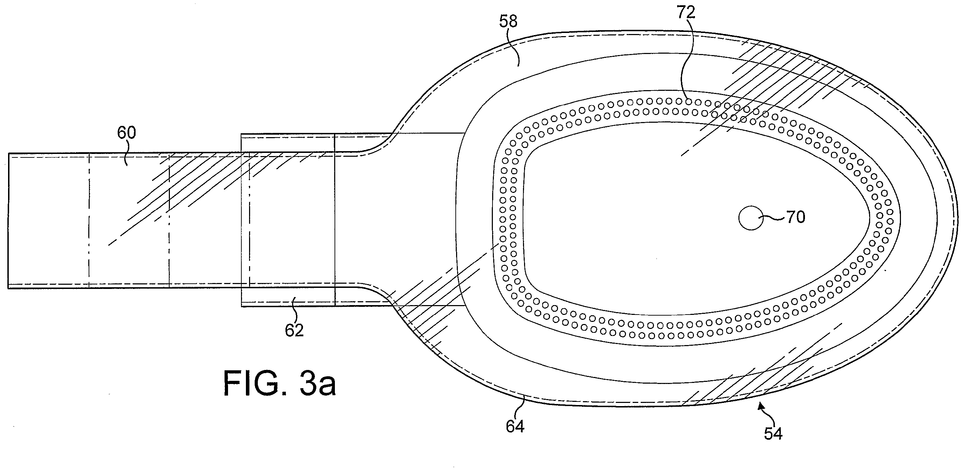

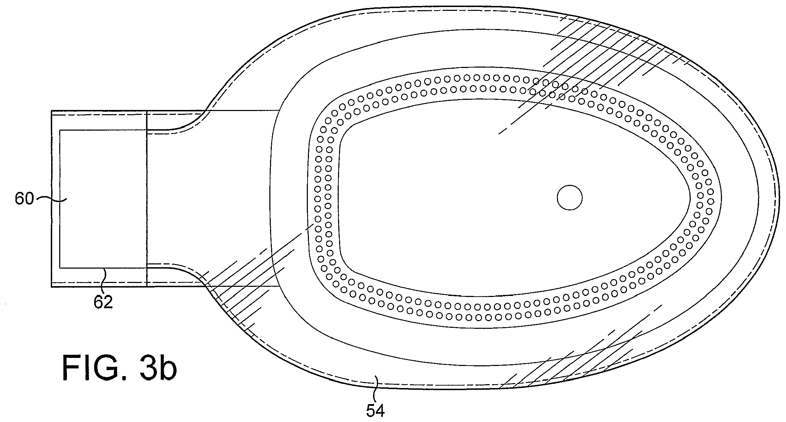

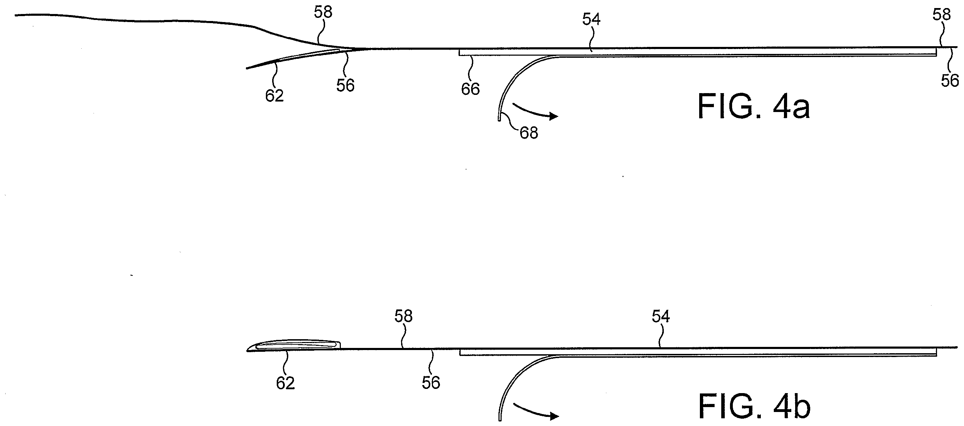

The known fistula bag 54 (an example only - the port is also suitable for use with other forms of appliance with different shape and/or dimensions) shown in Figures 3a to 4b is an EAKIN ® F12 fistula bag and includes two layers 56, 58 of thin membrane material which have a generally oval planform as shown in Figure 3a terminating at one end at a drainage tube 60 which may be extended as shown in Figure 3a or wrapped up and stowed in a sealing manner in a stowage pouch 62 as shown in Figure 3b. The membrane material layers 56, 58 are flexible and although shown as very close together and parallel in Figures 4a and 4b, due to the fact that the two membrane layers 56, 58 are only joined to one another at peripheral weld seam 64, the space between the two membranes 56, 58 may be inflated in to a bag shape, such as by the ingress of fluids into the bag 54.

Attached to the lower membrane surface 56 is a layer of COHESIVE ® sealing material 66 which is suitable for sealing the bag 54 onto a patient (not shown) once a peel strip 68 has been removed, thereby exposing the sealing material 66.

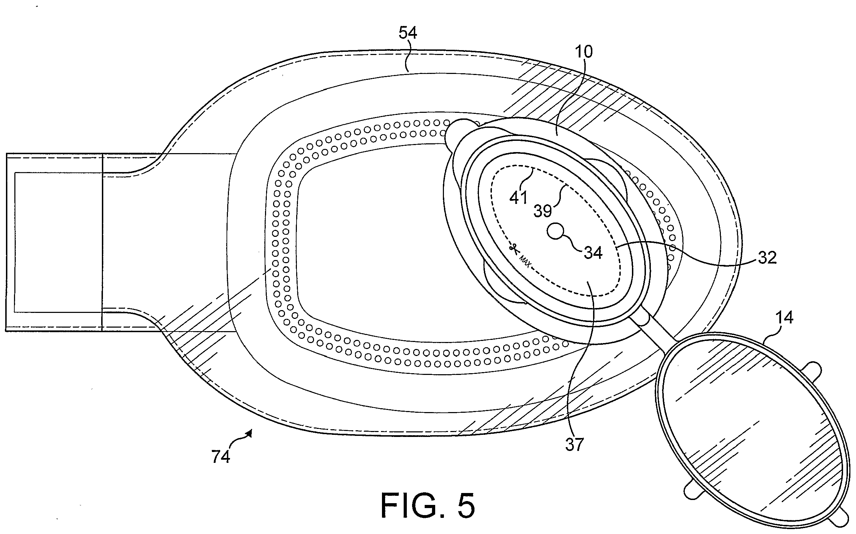

In use, a nurse or other operative may examine a patient's wound or fistula and select the bag 54. Next, starting at an access aperture which passes through sealing material 66 and lower layer 56 scissors may be used to enlarge the aperture to an appropriate size to surround the wound, but not to a larger size than a weld 72 which joins the sealing material 66 to the membrane sheet material 56 in a sealing manner. Next, the bag 54 may be laid on a flat surface (not shown). Next, the peel layer 28 may be removed, exposing the adhesive 26 which is a self-adhesive and covers the entire lower surface 24 of the mounting substrate 22. Next, the access port 10 may be positioned on the bag 54 wherever desired and pressed down as required to ensure a secure bond. The view in Figure 5 shows the access port 10 and bag 54 in this configuration with the lid 14 open. Next, the operative may using scissors or another suitable tool enlarge the optional aperture 34 by cutting through the mounting substrate 22 and membrane material 58 together to cut an enlarged access aperture of any convenient shape or size up to the border line of maximum extent shown by the indicia 32. As an alternative to enlargement of optional aperture 34 by cutting, aperture 34 may be absent and optional perforations 35 may be placed (for example at position of indicia 32) to allow the therapist or other user to push out and remove an inner part 37 of the substrate (or film) 22. Bag 54 may then be cut as desired to form an aperture in the membrane material 58, an example of whose location is shown in Figure 5 at the same position as aperture 41 to be formed by pushing out part 37 of substrate 22 defined by perforations 35. It will be appreciated that push out part 37 is shown for clarity in Figure 5 whereas in many cases it may actually be removed before applying port 10 to bag 54. Perforations may also extend through peel layer 28. The combined bag system 74 consisting of the assembled bag 54 and port 10 may then (after removing peel layer 68 and preparation of the bag 54 and layer 66 in accordance with manufacturer's instructions) be carefully pressed on to and sealed on to the patient using the adhesive layer 66. The lid 14 may then be closed and due to the transparent nature of the window 44 the wound or fistula, (not shown), if located below the window 44 or in the region thereof, may be easily inspected through the window 44. Also, due to the oval shape of the access port/ aperture 34 once enlarged up to or near its maximum extent, i.e. to the indicia line 32, access through the port 10 by hand is possible without making the overall size of the port 10 very large.

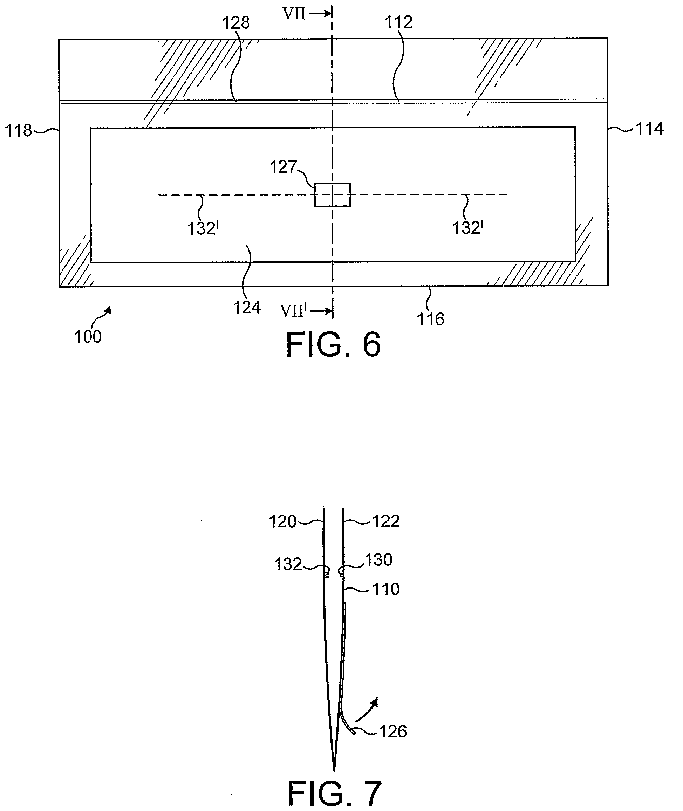

Figures 6 and 7 show another embodiment of an access port for use with the same type of bag as that shown in Figures 3a to 4b. The access port 100 shown in Figures 6 and 7 has a base 110 and a closure 112. The base consists of a flexible rectangular layer of membrane material welded at three edges thereof 114, 116, 118 to a similar layer of membrane material 120. The outer surface 122 of the base 110 has a rectangular self adhesive layer of material 124 thereon which is initially protected by a peel strip 126. The base 110 and adhesive layer 124 also have an optional rectangular access aperture 127 formed therein.

The closure 112 consists of an elongate fastener 128 joining the two edges 114, 118, the fastener 128 consisting of an elongate projection 130 on the base 110 which is adapted to releasably seal into elongate sealing channel 132 on the substrate 120.

The port 100 is used in a similar manner to the port 10 of Figure 1. The rectangular aperture 127 may be enlarged to form a larger access aperture or the user may stick port 100 on to the bag 54 and then enlarge the aperture 127 using scissors or the like, cutting simultaneously through the layer 58. Instead of enlarging the aperture 127 in that way, the user may simply decide to cut an elongate slit 132' through the base layer 110 and membrane material 58 to provide an elongate flexible access port. Slit 132' may be pre-formed by the manufacturer, such as in cases when optional aperture 127 is absent.
The embodiments described in these and other preferred embodiments provide a medical bag system with an access port with a self-adhesive layer which allows the port to be positioned in an ideal place on a pouch, bag or other medical appliance by a therapist or nurse. The access ports allow access to wounds, fistulas or stomas by therapists/nurses or other operators to observe/clean and treat the same either by looking through the window 44 or transparent base 110 and layer 120, or by opening the closure, i.e. the lid 14 or the closure 112. The therapist or nurse may install the access port on almost any type of plastic-based appliance that would benefit from an access port in a medical environment. The access port may be supplied in a range of sizes such that larger or smaller access ports than those shown in Figures 1 and 6 may be provided but having the same or similar overall configuration. The opening 34, 132', 127 may be made large enough to accommodate a hand for cleaning and treatment purposes. In the example shown in Figure 5 the access aperture 34 once enlarged to the indicia 35 may have a cross-dimension along the short access of the port of about 5 to 7 cm and a cross-dimension along the long axis of the port of about 8 to 11 cm, thus allowing easy access by hand, tool or other media through the port for patient therapy.

It is envisaged that in some cases port 10, 100 may be used with a bag or other medical appliance and in a method of operation intended to and actually producing a solely cosmetic effect, such as a beauty treatment for skin (e.g. tanning), hair (e.g. tinting) or the like. It is also envisaged that the invention extends to a medical bag system with an access port and methods of operation used in relation to deceased humans or animals.
Apart from the peel layer 28, 126 the materials in the port 10, 100 may all be plastics and relatively flexible. However, a reliable seal is needed between the outer edge 14 and rim 18, as well as between the corporating closure parts 130, 132.
Various modifications may be made to the embodiments described without departing from the scope of the invention as defined by the accompanying claims.

## Claims

1. A medical bag system comprising a medical bag (54) and a therapy access port (10, 100);
said medical bag (54) having a membrane surface (56) on a first side thereof, and in which the membrane surface (56) is arranged for the formation of an inspection hole (70) therein;
said therapy access port (10, 100) configured for allowing therapy of a patient therethrough, the port comprising a base (12, 110) having a generally flat base plate (16) having an access aperture (34) formed in the centre thereof, an openable closure (14, 112) for the base (12, 110), and wherein the access port comprises a seal (26, 124), **characterised in that** the seal comprises an adhesive layer (26) adjacent the base plate, the adhesive layer (26) being located upon and covering an area of a mounting substrate (22), the mounting substrate (22) being secured to the base plate (16), the adhesive layer (26) being on a surface (24) of the substrate (22) facing away from the base plate (16), which adhesive layer (26) in a pre-used configuration is protected by a peel off layer (28), wherein said medical bag is configured and said seal (26, 124) is arranged to be located on the base (12, 110) for sealingly connecting the port (10, 100) to said medical bag (54) in one of a choice of locations on said medical bag.

2. A medical bag system as claimed in claim 1 in which the bag (54) is arranged to be mounted on a patient.

3. A medical bag system as claimed in claim 1 or 2, wherein the bag (54) has a sealing layer (66) on a second side thereof opposite the first side which is adapted to seal the bag (54) to a patient.

4. A medical bag system as claimed in any one of the preceding claims, wherein the base (12) and/or the access port has a non-circular yet curved, preferably oval, configuration.

5. A medical bag system as claimed in any preceding claim in which the base (12) has a generally flat base plate (16) having an access aperture (34) formed in the centre thereof.

6. A medical bag system as claimed in any one of the preceding claims, wherein the mounting substrate (22) includes perforations (35) therethrough enabling the pre-defined part of the substrate (22) to be removed by tearing the substrate (22).

7. A medical bag system as claimed in any preceding claim in which the closure member (14) has an outer edge (38) arranged to snap-lockingly seal onto a connector part (18) of the base plate (12).

8. A medical bag system as claimed in any one of claims 1 to 7 in which the access port (100) comprises an access bag and the base plate comprises a first flexible substrate layer (110) of the access bag.

9. A medical bag system as claimed in claim 8 when dependent on claim 7 in which the closure (112) comprises a second flexible substrate layer (120) wherein the flexible substrate layers (110, 120) are joined at respective edges (114, 116, 118) thereof, and at least one elongate component (128) arranged to releasably join the first and second flexible substrate layers (110, 120) together therealong for releasably closing the access bag.

10. A medical bag system as claimed in claim 9, wherein the first flexible substrate layer (110) has a slit (132') formed therethrough for communication with the interior of a medical bag (54).

11. A method of assembling the medical bag system as claimed in any one of claims 1 to 10, the method comprising selecting a desired location for mounting the therapy access port (10, 100) on the medical bag and mounting the access port (10, 100) on the medical bag in the desired location with an adhesive component (26, 124).

12. A method as claimed in claim 11 which includes forming a communication aperture in the bag (54) once the port (10, 100) is mounted on the bag.

## Patentansprüche

1. Medizinisches Beutelsystem, umfassend einen medizinischen Beutel (54) und einen Therapiezugangsanschluss (10,100);
wobei der medizinische Beutel (54) eine Membranoberfläche (56) an einer ersten Seite davon aufweist, wobei die Membranoberfläche (56) zum Bilden eines Beobachtungslochs (70) darin beschaffen ist;
wobei der Therapiezugangsanschluss (10,100) dafür gestaltet ist, durch ihn hindurch Therapie eines Patienten zu ermöglichen, wobei der Anschluss eine Basis (12, 110) mit einer allgemein flachen Basisplatte (16), die eine in ihrer Mitte gebildete Zugangsöffnung (34) aufweist, und einen öffnungsfähigen Verschluss (14, 112) für die Basis (12, 110) umfasst, wobei die Zugangsöffnung eine Dichtung (26, 124) umfasst, **dadurch gekennzeichnet, dass** die Dichtung eine Klebstoffschicht (26) benachbart zu der Basisplatte umfasst, wobei die Klebstoffschicht (26) auf einer Fläche eines Befestigungssubstrats (22) angeordnet ist und sie bedeckt, wobei das Befestigungssubstrat (22) an der Basisplatte (16) befestigt ist, wobei die Klebstoffschicht (26) an einer Oberfläche (24) des Substrats (22) angeordnet ist, die von der Basisplatte (16) weg zeigt, welche Klebstoffschicht (26) in einer Konfiguration vor der Verwendung von einer Abziehschicht (28) geschützt ist, wobei der medizinische Beutel dafür gestaltet ist und die Dichtung (26, 124) dafür beschaffen ist, an der Basis (12, 110) angeordnet zu werden, um den Anschluss (10, 100) an einem von einer Auswahl von Orten an dem medizinischen Beutel abdichtend mit dem medizinischen Beutel (54) zu verbinden.

2. Medizinisches Beutelsystem gemäß Anspruch 1, wobei der Beutel (54) zum Anbringen an einem Patienten beschaffen ist.

3. Medizinisches Beutelsystem gemäß Anspruch 1 oder 2, wobei der Beutel (54) eine Dichtungsschicht (66) an einer zweiten Seite davon aufweist, die der ersten Seite gegenüberliegt, die zum Anbringen des Beutels (54) an einen Patienten ausgelegt ist.

4. Medizinisches Beutelsystem gemäß einem der vorstehenden Ansprüche, wobei die Basis (12) und/oder der Zugangsanschluss eine nichtkreisförmige aber gekrümmte, vorzugsweise ovale, Konfiguration aufweist.

5. Medizinisches Beutelsystem gemäß einem der vorstehenden Ansprüche, wobei die Basis (12) eine allgemein flache Basisplatte (16) aufweist, die eine Zugangsöffnung (34) in ihrer Mitte gebildet aufweist.

6. Medizinisches Beutelsystem gemäß einem der vorstehenden Ansprüche, wobei das Befestigungssubstrat (22) hindurchgehende Perforationen (35) aufweist, die erlauben, dass ein vordefinierter Teil des Substrats (22) durch Reißen des Substrats (22) entfernt werden kann.

7. Medizinisches Beutelsystem gemäß einem der vorstehenden Ansprüche, wobei das Verschlusselement (14) einen Außenrand (38) aufweist, der dafür beschaffen ist, durch Schnappverriegelung gegenüber einem Verbindungsteil (18) der Basisplatte (12) abgedichtet zu werden.

8. Medizinisches Beutelsystem gemäß einem der Ansprüche 1 bis 7, wobei der Zugangsanschluss (100) einen Zugangsbeutel umfasst und die Basisplatte eine erste flexible Substratschicht (110) des Zugangsbeutels umfasst.

9. Medizinisches Beutelsystem gemäß Anspruch 8, wenn abhängig von Anspruch 7, wobei der Verschluss (112) eine zweite flexible Substratschicht (120) umfasst, wobei die flexiblen Substratschichten (110, 120) an entsprechenden Rändern (114, 116, 118) davon verbunden sind, und wenigstens eine langgestreckte Komponente (128), die dafür beschaffen ist, an ihr entlang die erste und die zweite flexible Substratschicht (110, 120) lösbar zu verbinden, um den Beutel lösbar zu verschließen.

10. Medizinisches Beutelsystem gemäß Anspruch 9, wobei die erste flexible Substratschicht (110) einen hindurchgehenden Schlitz (132') zur Kommunikation mit dem Innenraum eines medizinischen Beutels (54) aufweist.

11. Verfahren zum Zusammensetzen des medizinischen Beutelsystems gemäß einem der Ansprüche 1 bis 10, wobei das Verfahren Auswählen einer gewünschten Stelle zum Befestigen des Therapiezugangsanschlusses (10, 100) an dem medizinischen Beutel und Befestigen des Zugangsanschlusses (10, 100) an dem medizinischen Beutel an dem gewünschten Ort mit einer Haftkomponente (26, 124) umfasst.

12. Verfahren gemäß Anspruch 11, umfassend Bilden einer Verbindungsöffnung in dem Beutel (54), sobald der Anschluss (10, 100) an dem Beutel befestigt ist.

## Revendications

1. Système de sachet médical comprenant un sachet médical (54) et un orifice (10, 100) d'accès thérapeutique,
ledit sachet médical (54) comportant une surface (56) de membrane sur un premier côté de celui-ci, et dans lequel la surface (56) de membrane est agencée pour y former un trou d'inspection (70) ;
ledit orifice (10, 100) d'accès thérapeutique étant configuré pour permettre de soigner un patient à travers lui, l'orifice comprenant une base (12, 110) comportant une plaque (16) de base dans l'ensemble plate ayant une ouverture d'accès (34) pratiquée en son centre et une fermeture (14, 112) pouvant s'ouvrir pour la base (12, 110), et dans lequel l'orifice d'accès comprend un joint d'étanchéité (26, 124),
**caractérisé en ce que** le joint comprend une couche adhésive (26) adjacente à la plaque de base, la couche adhésive (26) étant située sur une zone d'un substrat de montage (22) et la couvrant, le substrat de montage (22) étant bien fixé à la plaque (16) de base, la couche adhésive (26) étant sur la surface (24) du substrat (22) tournée à l'opposé de la plaque (16) de base, laquelle couche adhésive (26), dans une configuration avant utilisation, est protégée par un endos détachable (28), dans lequel ledit sachet médical est configuré et ledit joint d'étanchéité (26, 124) est agencé pour être situés sur la base (12, 110) afin de raccorder de façon étanche l'orifice (10, 100) audit sachet médical (54) dans un des emplacements choisis sur ledit sachet médical.

2. Système de sachet médical selon la revendication 1, dans lequel le sachet (54) est agencé pour être installé sur un patient.

3. Système de sachet médical selon la revendication 1 ou 2, dans lequel le sachet (54) comporte sur son second côté à l'opposé du premier côté une couche de collage (66) qui est apte à coller le sachet (54) sur un patient.

4. Système de sachet médical selon l'une quelconque des revendications précédentes, dans lequel la base (12) et/ou l'orifice d'accès ont une configuration non circulaire mais néanmoins courbe, de préférence ovale.

5. Système de sachet médical selon l'une quelconque des revendications précédentes, dans lequel la base (12) comporte une plaque (16) de base dans l'ensemble plate ayant une ouverture d'accès (34) pratiquée en son centre.

6. Système de sachet médical selon l'une quelconque des revendications précédentes, dans lequel le substrat de montage (22) comprend des perforations (35) qui le traversent et permettent d'enlever la partie prédéfinie du substrat (22) en déchirant le substrat (22).

7. Système de sachet médical selon l'une quelconque des revendications précédentes, dans lequel l'élément de fermeture (14) comporte un bord extérieur (38) agencé pour se fermer hermétiquement et automatiquement sur la partie de connecteur (18) de la plaque de base (12).

8. Système de sachet médical selon l'une quelconque des revendications 1 à 7, dans lequel l'orifice d'accès (100) comprend un sachet d'accès et la plaque de base comprend une première couche (110) flexible de substrat du sachet d'accès.

9. Système de sachet médical selon la revendication 8 quand elle est subordonnée à la revendication 7, dans lequel la fermeture (112) comprend :
une seconde couche (120) flexible de substrat, dans lequel les couches (110, 120) flexibles de substrat sont assemblées au niveau de leurs bords (114, 116, 118) respectifs, et
au moins un composant (128) allongé, agencé pour assembler sur sa longueur, de façon amovible les première et seconde couches (110, 120) flexibles de substrat afin de fermer le sachet d'accès avec réouverture possible.

10. Système de sachet médical selon la revendication 9, dans lequel la première couche (110) flexible de substrat comporte une fente (132') qui la traverse pour communiquer avec l'intérieur d'un sachet médical (54).

11. Procédé d'assemblage du système de sachet médical selon l'une quelconque des revendications 1 à 10, le procédé comprenant les opérations consistant à choisir un emplacement souhaité pour le montage de l'orifice (10, 100) d'accès thérapeutique sur le sachet médical et monter l'orifice (10, 100) d'accès sur le sachet médical à l'emplacement souhaité avec un composant adhésif (26, 124) .

12. Procédé selon la revendication 11, qui comprend l'opération consistant à pratiquer une ouverture de communication dans le sachet (54) une fois que l'orifice (10, 100) est monté sur le sachet.
